# EUROPÄISCHE PATENTANMELDUNG

(11) **EP 1 156 440 A2**
(43) Veröffentlichungstag der Anmeldung: **21.11.2001**
(21) Anmeldenummer: 01108758.2
(22) Anmeldetag: 06.04.2001
(51) Int. Cl.: G06F 19/00

(54) **Verfahren und Vorrichtung zur automatisierten Aufdeckung gesundheitlicher Risken für einen Patienten**

(30) Priorität: 19.04.2000 JP 1001009498
(71) Anmelder: SIEMENS AKTIENGESELLSCHAFT, 80333 München (DE)
(72) Erfinder: Abraham-Fuchs, Klaus, 91058 Erlangen (DE)

(57) **Zusammenfassung**

Verfahren zur automatisierten Aufdeckung gesundheitlicher Risiken für einen Patienten unter Verwendung einer elektronischen Datenbank für Patientendaten (EPR) und eines Expertensystems, das mittels implementierter medizinischer Regeln aus einer Kombination mehrerer Daten eine Verdachtsdiagnose oder ein erhöhtes Krankheitsrisiko ableiten kann, wobei durch jede Eingabe neuer Daten in die EPR diese neuen Daten gleichzeitig zusammen mit allen bereits abgespeicherten Daten dieses Patienten dem dabei gestarteten Expertensystem zugespielt werden, das bei geänderter Risikobeurteilung eine Meldung an die Eingabestelle, den Patienten oder den behandelnden Arzt abgibt.

## Beschreibung

Die Erfindung bezieht sich auf ein Verfahren zur automatisierten Aufdeckung gesundheitlicher Risiken für einen Patienten unter Verwendung einer elektronischen Datenbank für Patientendaten (EPR) und eines Expertensystems, das mittels implementierter medizinischer Regeln aus einer Kombination mehrerer Daten eine Verdachtsdiagnose oder ein erhöhtes Krankheitsrisiko ableiten kann, sowie eine Vorrichtung zur Durchführung dieses Verfahrens.

Die heutigen Gesundheitssysteme, speziell in den hochindustrialisierten Ländern der Welt, sind durch eine ausgeprägte Rollenverteilung und starke Spezialisierung der Ärzte geprägt. Dadurch wird medizinisch relevante Information an vielen verschiedenen Daten und zu unterschiedlichsten Zeitpunkten erhoben. Es kommt daher häufig vor, dass einem gerade behandelnden Arzt die Summe aller derjenigen medizinischen Informationen, die zur Diagnose einer Krankheit oder der Erkennung eines behandlungsbedürftigen erhöhten Risikozustandes eines Patienten führt, nicht zur Verfügung stehen. Die Krankheit wird daher übersehen, obwohl die notwendigen Informationen zur Erkennung der Krankheit woanders vorliegen würden. Ein wichtiger Schritt, diesen Missstand abzuhelfen, ist die durch moderne I&K-Technologien möglich gewordene Einrichtung einer "Elektronischen Patientenakte". Eine mögliche Implementierung einer EPR ist z. B. die, alle medizinisch relevanten Daten am Ort der Erhebung (Arztpraxis, Krankenhaus usw.) zu speichern und diese Information durch Vernetzung mit einem zentralen Server anderen Berechtigten an jedem Ort und zu jeder Zeit verfügbar zu machen. Dadurch ist zwar für einen Patienten bei dem ihn gerade behandelnden Arzt jetzt theoretisch alle Information verfügbar. Die Informationsmenge wird jetzt aber viel zu groß sein, als dass der Arzt bei einem neuen, für sich allein unverdächtigen Messwert die gesamte Information der Datenbank zu Rate ziehen kann, um einen erst durch die Verknüpfung mit alten Informationen sich ergebenden Warnhinweis zu erkennen.

Ein eingangs genanntes Verfahren und eine Vorrichtung zur Durchführung des Verfahrens ist aus der US 5,517,405 bekannt. Dort ist ein computergestütztes Entscheidungssystem beschrieben, das einem Benutzer ermöglicht zu entscheiden, ob er eine vorgeschlagene Lösung eines Problems akzeptiert oder zurückweist. Nach einer Anfrage an das System, wobei Beschwerden des Patienten beschreibende Daten eingegeben werden, ermittelt das System, welche tatsächlichen Ursachen seine Beschwerden haben und gibt Behandlungsvorschläge aus.

Der Erfindung liegt daher die Aufgabe zugrunde, neben der Speicherung der Daten und dem Management des Datenflusses eine automatisierte Sichtung und Bewertung der Datenmengen zu erzielen.

Zur Lösung dieser Aufgabe ist erfindungsgemäß vorgesehen, dass durch jede Eingabe neuer Daten in die EPR diese neuen Daten gleichzeitig zusammen mit allen bereits abgespeicherten Daten dieses Patienten dem dabei gleichzeitig gestarteten Expertensystem zugespielt werden, das bei geänderter Risikobeurteilung eine Meldung z. B. an die Eingabestelle, den Patienten oder den behandelnden Arzt abgibt. Diese Meldung kann dabei gleichzeitig mit einer entsprechenden Handlungsaufforderung - z. B. "gehen Sie zu Ihrem Arzt, um die Untersuchung X durchführen zu lassen" oder "Ihre Lebensumstände zeigen ein deutlich erhöhtes Risiko für die Krankheit Y. Auf der Webside Z können Sie Informationen über diese Krankheit und mögliche Vorbeugungsmaßnahmen finden." - gegebenenfalls auch zusätzliche Therapievorschläge unterbreiten.

Gemäß der vorliegenden Erfindung erfolgt also eine besondere Verknüpfung der Patientendaten in der EPR mit einem Expertensystem dergestalt, dass automatisch bei jeder neuen Dateneingabe zu einem Patienten dessen Altdaten mit den neuen Daten zusammen dem Expertensystem zur Verfügung gestellt werden, das gleichzeitig gestartet wird, um die eingespielten Patientendaten - gegebenenfalls kann durch das Starten auch ein selbsttätiger Zugriff des Expertensystems auf die gespeicherten Daten erfolgen - automatisch neu zu bewerten und zu ermitteln, ob durch die neuen Eingabedaten eine neue Krankheit oder ein erhöhtes Risiko für eine Erkrankung festgestellt werden kann. Ist dies nicht der Fall, so schaltet sich das Expertensystem automatisch ab. Wenn aber eine geänderte Risikobeurteilung vorliegt, so gibt es Meldung an unterschiedliche Empfängerstellen, also insbesondere dem Patienten oder dessen Arzt.

Zur Durchführung des erfindungsgemäßen Verfahrens ist vorgesehen, dass einer elektronischen Datenbank für Patientendaten (EPR) mit wenigstens einem Eingabeterminal und einem Expertensystem, z. B. in Form eines Bayes'schen Netzwerks oder eines Fuzzy-Logic-Algorithmus, ein Verknüpfungssystem zugeordnet ist, das bei Betätigung eines Eingabeterminals das Expertensystem startet und ihm die Eingabedaten und alle Speicherdaten des Patienten zur Verfügung stellt.

Das mit vorgebbaren Empfängern verbundene Expertensystem kann dabei mit besonderem Vorteil im zentralen Server des EPR integriert sein, wobei das Verknüpfungssystem so ausgebildet sein soll, dass es die gleichzeitige Erfassung aller gespeicherten Patientendaten auch bei dezentralem Aufbau der EPR ermöglicht.

Weitere Vorteile, Merkmale und Einzelheiten der Erfindung ergeben sich aus der nachfolgenden Beschreibung eines Ausführungsbeispiels sowie anhand der Zeichnung. Dabei zeigen:
- Fig. 1: ein Flussdiagramm des Ablaufs des erfindungsgemäßen Verfahrens zur automatisierten Aufdeckung gesund- heitlicher Risiken eines Patienten und
- Fig. 2: ein schematisches Blockdiagramm des Aufbaus der Vorrichtung zur Durchführung dieses Verfahrens.

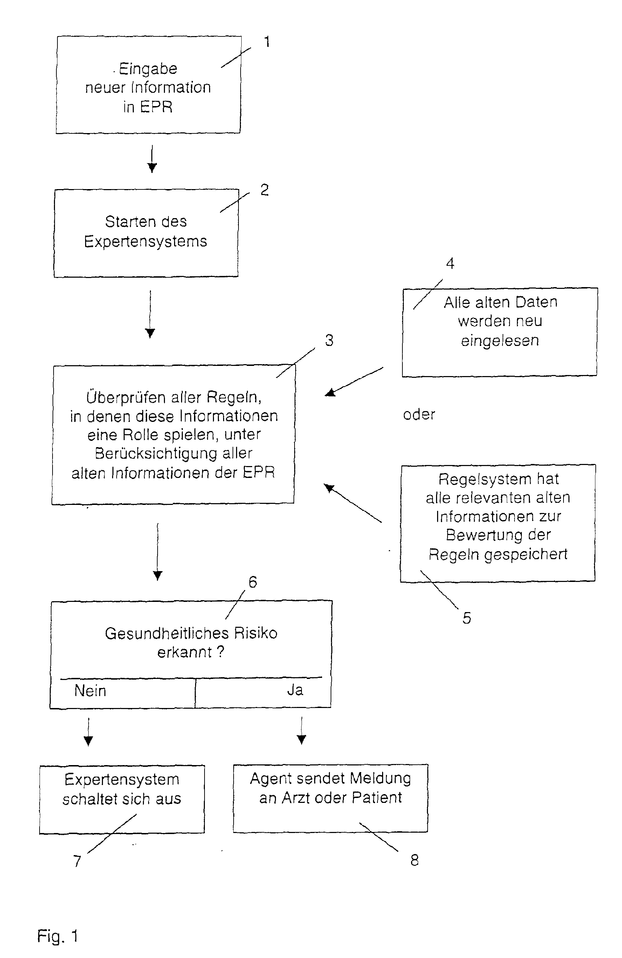

In der Stufe 1 des in Fig. 1 dargestellten Ablaufdiagramms erfolgt eine Eingabe neuer Informationen in eine elektronische Datenbank für Patientendaten. Dadurch wird in Stufe 2 automatisch ein mit der EPR verknüpftes Expertensystem gestartet, das eine Überprüfung aller Regeln, in denen diese Informationen eine Rolle spielen, unter Berücksichtigung aller alten Informationen in der EPR durchführt. Um diese alten Informationen mit zu berücksichtigen, werden sie entweder gemäß Verfahrensstufe 4 mit dem Starten es Expertensystem in dieses eingelesen oder aber das Regelsystem ist so in das EPR integriert, dass ihm diese alten Informationen direkt zur Verfügung stehen (Verfahrensablaufstufe 5 gemäß Fig. 1). Das Expertensystem erstellt aus all diesen Daten eine Neubeurteilung bezüglich eines gesundheitlichen Risikos in Stufe 6, wobei bei Nichtdiagnostizierung neuer Risiken oder Krankheiten das Expertensystem gemäß Stufe 7 sich selbsttätig wieder abschaltet oder aber gemäß Stufe 8 eine Meldung an den Arzt oder den Patienten absendet.

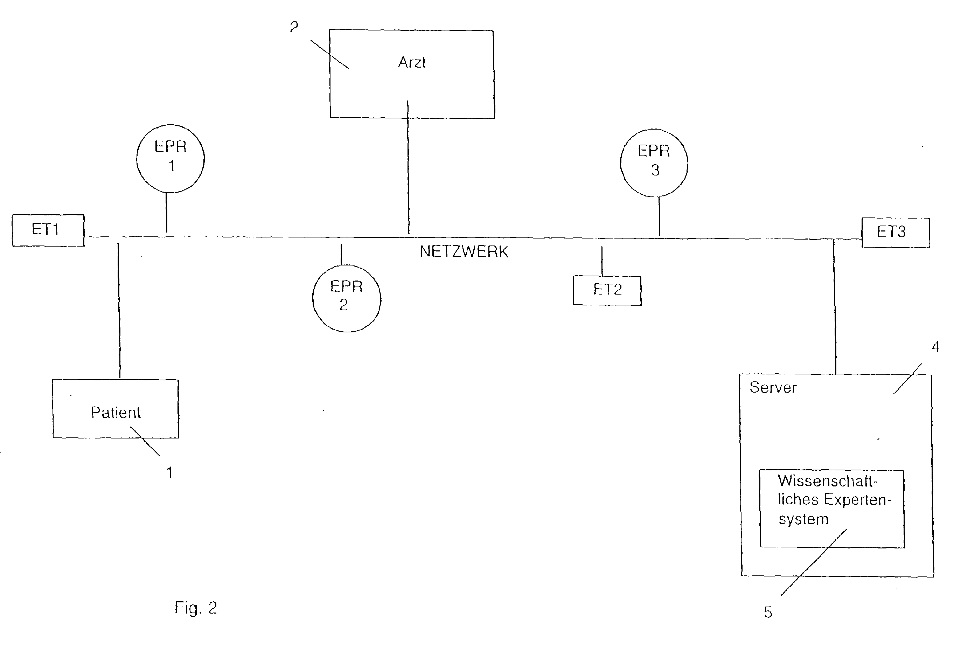

Die Fig. 2 zeigt schematisch das System zur Durchführung des erfindungsgemäßen Verfahrens, bei dem der Patient 1 und der Arzt 2 sowie gegebenenfalls einige zusätzliche Eingabeterminals ET1, ET2 und ET3 über ein Netzwerk 3 mit einer elektronischen Datenbank für Patientendaten verbunden sind, die in der schematischen Darstellung nach Fig. 2 durch die drei EPR1, EPR2 und EPR 3 angedeutet sind. Ein Server 4 sorgt für die Verknüpfung der Eingabeterminals des Patienten, des Arztes und der elektronischen Datenbank für Patientendaten über das Netzwerk 3 miteinander sowie mit einem wissenschaftlichen Expertensystem 5, das im dargestellten Ausführungsbeispiel in den Server 4 integriert ist, jedoch selbstverständlich auch an anderer Stelle angeordnet sein könnte.

Die Erfindung ist nicht auf das dargestellte Ausführungsbeispiel beschränkt. Die Art und Weise der Verknüpfung der Eingabestellen mit dem EPR und dem Expertensystem sowie die unterschiedlichen Möglichkeiten einer Rückkopplung könnten auch in anderer Weise realisiert sein. Entscheidend für die vorliegenden Erfindung ist die automatische Inanspruchnahme eines Expertensystem bei jeder neuen Patientendateneingabe unter gleichzeitiger Heranziehung der alten gespeicherten Patientendaten.

## Patentansprüche

1. Verfahren zur automatisierten Aufdeckung gesundheitlicher Risiken für einen Patienten unter Verwendung einer elektronischen Datenbank für Patientendaten (EPR) und eines Expertensystems, das mittels implementierter medizinischer Regeln aus einer Kombination mehrerer Daten eine Verdachtsdiagnose oder ein erhöhtes Krankheitsrisiko ableiten kann,
**dadurch gekennzeichnet, dass** durch jede Eingabe neuer Daten in die EPR diese neuen Daten gleichzeitig zusammen mit allen bereits abgespeicherten Daten dieses Patienten dem dabei gestarteten Expertensystem zugespielt werden, das bei geänderter Risikobeurteilung eine Meldung z. B. an die Eingabestelle, den Patienten oder den behandelnden Arzt abgibt.

2. Verfahren nach Anspruch 1, **dadurch gekennzeichnet, dass** das Expertensystem gleichzeitig einen Therapie- oder Untersuchungsvorschlag unterbreitet.

3. Vorrichtung zur Durchführung des Verfahrens nach Anspruch 1 oder 2, **dadurch gekennzeichnet, dass** einer EPR mit wenigstens einem Eingabeterminal und einem Expertensystem (z. B. in Form eines Bayes'schen Netzwerks oder eines Fuzzy-Logic-Algorithmus) ein Verknüpfungssystem zugeordnet ist, das bei Betätigung eines Eingabeterminals das Expertensystem startet und ihm die Eingabedaten und alle Speicherdaten des jeweiligen Patienten zur Verfügung stellt.

4. Vorrichtung nach Anspruch 3, **dadurch gekennzeichnet, dass** das Expertensystem im zentralen Server des EPR integriert ist.

5. Vorrichtung nach Anspruch 3 oder 4, das Verknüpfungssystem so ausgebildet ist, dass es die gleichzeitige Erfassung aller gespeicherten Patientendaten auch bei dezentralen Aufbau der EPR ermöglicht.
